# EUROPEAN PATENT APPLICATION

(11) **EP 3 832 572 A1**
(43) Date of publication of application: **09.06.2021**
(21) Application number: 20204717.1
(22) Date of filing: 24.07.2015
(51) Int. Cl.: G06Q 10/10, G06Q 30/00, H04W 4/12, G06Q 50/22

(54) **RENEWAL MESSAGE SYSTEM**

(30) Priority: 28.07.2014 GB 201413338
(62) Divisional of application: 15756429.5
(71) Applicant: Virtual Recall Limited, Guildford, Surrey GU2 7RE (GB)
(72) Inventor: Crittall, James, Guildford, Surrey GU4 8DD (GB); Barton, Charles, Bramerton Norwich NR14 7EG (GB)
(74) Representative: Fry, David John

(57) **Abstract**

A system for generating renewal messages for transmitting to remote customers is disclosed. The system comprises at least one memory, storing: for each of a plurality of products, a renewal profile indicating at least a renewal period for the product; and customer data, including SMS contact data and one or more product(s) associated with the customer including an associated reference date. A processor is configured automatically to: generate based on the current date, the renewal profile for each product and the reference date(s) a list of one or more customers for whom a renewal message is required; and generate a single message for the or each customer, which message references multiple product renewals and is formatted to be appropriate to sending as a single SMS message.

## Description

### Field of the Invention

This invention relates to a renewal message system and method, for example for generating reminders relating to renewing a product or service.

### Background of the Invention

In many situations, a product or service needs to be renewed or re-performed periodically. One example is a vehicle service, which is typically performed annually. Another example is the re-ordering of medicines or treatments from a doctor or veterinary surgeon, the renewal period for which can vary depending from product to product.

Typically, it is left to the customer to note the renewal date, or, as in the case of medicines, to visually note when the current supply is running low. This means that customers may be adversely affected if they pass a renewal date, e.g. if they miss a service deadline, their vehicle warranty may expire, or in the case of medicines, they may be without their treatment for a period of time.

It would be desirable to offer some form of automated reminder system that can warn a customer in advance of an upcoming renewal date.

In the following, and for the avoidance of doubt, references to a product include a service also.

### Summary of the Invention

A first aspect of the invention provides a system for generating renewal messages for transmitting to remote customers, the system comprising: at least one memory, storing: for each of a plurality of products, a renewal profile indicating at least a renewal period for the product; customer data, including SMS contact data and one or more product(s) associated with the customer including an associated reference date; a processor configured automatically to: generate based on the current date, the renewal profile for each product and the reference date(s) a list of one or more customers for whom a renewal message is required; and generate a single message for the or each customer, which message references multiple product renewals and is formatted to be appropriate to sending as a single SMS message.

The system may further comprise a SMS gateway or server configured automatically to send the or each single message generated using the customer's contact data.

The processor may be configured to periodically generate the list and message at substantially the same time of day.

The renewal profile for a product stored in the at least one memory may comprise:
- a product identifier;
- a renewal period; and
- an advance period,
and wherein the processor in generating the list of customers may be configured to perform the steps of, for each product, using the renewal period and advance period for the product to determine a comparison reference date; identifying from the customer data those customers having the associated product identifier and a match between the comparison references date and the customer reference date, whereafter matches are added to the customer list, including the relevant product identifier.

The customer data reference date may be a purchase/invoice date for the product stored on an electronic records/invoicing system. The purchase/invoice date may be received from a different computer program which is independent of that used to generate renewal messages. The purchase/invoice date may be received from an external computer system. The customer data reference date may be received by initiating a periodic data request to an external computer program or system.

The system may further comprise providing a SMS message template comprising a standard message and one or more data entry fields into which the processor automatically populates renewal data.

A plurality of templates may be provided dependant on context, e.g. number of renewals.

The system may be a medical or veterinary product reminder system for issuing renewal reminders to patient or animal owners. The system may relate to other applications, such as vehicle servicing reminders, MOT renewals, contact lens renewals, swimming pool treatment renewals and so on.

The memory may further store, as part of, or associated with, the customer data, the identity of a storage container associated with one or more respective customers, and is configured responsive to receiving an electronic message initiated by said storage container to include in said generated message a reference to a product renewal, which product is associated with the user or storage container.

The system may further configured to receive a pairing message received from a remote location containing an indication of a customer identifier and the storage container identifier, and to update the customer data automatically to include the storage container identifier.

The pairing message may further contain a product identifier or code, which is updated to the customer data or associated with it.

A second aspect of the invention provides a method for generating renewal messages for transmitting to remote customers, the method comprising:
(1) storing:
   (i) for each of a plurality of products, a renewal profile indicating at least a renewal period for the product;
   (ii) customer data, including SMS contact data and one or more product(s) associated with the customer including an associated reference date;
(2) generating based on the current date, the renewal profile for each product and the reference date(s) a list of one or more customers for whom a renewal message is required; and
(3) generating a single message for the or each customer, which message references multiple product renewals and is formatted to be appropriate to sending as a single SMS message.

The method may further comprise using a SMS gateway or server automatically to send the or each single message generated using the customer's contact data.

The method may further comprise periodically generating the list and message at substantially the same time of day.

The renewal profile for a product may comprise:
- a product identifier;
- a renewal period; and
- an advance period,
and wherein generating in step (2) comprises, for each product,
- using the renewal period and advance period for the product to determine a comparison reference date; and
- identifying from the customer data those customers having the associated product identifier and a match between the comparison references date and the customer reference date, whereafter matches are added to the customer list, including the relevant product identifier.

The customer data reference date may be a purchase/invoice date for the product stored on an electronic records/invoicing system. The purchase/invoice date may be received from a different computer program which is independent of that used to generate renewal messages. The purchase/invoice date may be received from an external computer system. The customer data reference date may be received by initiating a periodic data request to an external computer program or system.

The method may further comprise providing a SMS message template comprising a standard message and one or more data entry fields into which the processor automatically populates renewal data.

A plurality of templates may be provided, dependant on context, e.g. number of renewals.

A third aspect of the invention provides a method of generating medical or veterinary product reminders using the method of any preceding definition.

A fourth aspect of the invention provides a computer program comprising instructions that when executed by a computer apparatus control it to perform the method of any preceding definition.

A fifth aspect of the invention provides a non-transitory computer-readable storage medium having stored thereon computer-readable code, which, when executed by computing apparatus, causes the computing apparatus to perform a method comprising:
(1) storing:
   (i) for each of a plurality of products, a renewal profile indicating at least a renewal period for the product;
   (ii) customer data, including SMS contact data and one or more product(s) associated with the customer including an associated reference date;
(2) generating based on the current date, the renewal profile for each product and the reference date(s) a list of one or more customers for whom a renewal message is required; and
(3) generating a single message for the or each customer, which message references multiple product renewals and is formatted to be appropriate to sending as a single SMS message.

A sixth aspect of the invention provides an apparatus, the apparatus having a processing system having one or more processors and at least one memory having computer-readable code stored thereon which when executed controls the processing system:
(1) to store:
   (i) for each of a plurality of products, a renewal profile indicating at least a renewal period for the product;
   (ii) customer data, including SMS contact data and one or more product(s) associated with the customer including an associated reference date;
(2) to generate based on the current date, the renewal profile for each product and the reference date(s) a list of one or more customers for whom a renewal message is required; and
(3) to generate a single message for the or each customer, which message references multiple product renewals and is formatted to be appropriate to sending as a single SMS message.

### Brief Description of the Drawings

The invention will now be described by way of non-limiting example with reference to the accompanying drawings, in which:
Figure 1 is a block diagram of a renewal reminder system according to the invention, used in a practical situation;
Figure 2 is a schematic block diagram of hardware components of the renewal reminder system in Figure 1;
Figure 3 is a schematic block diagram of functional modules of the renewal reminder system in Figure 1 and Figure 2;
Figure 4 is a flow chart of processing steps performed by the renewal reminder system for generating client reminder messages and sending them to remote locations;
Figure 5 is a flow chart of processing steps performed by the renewal reminder system in generating a reminder table, which forms part of the Figure 4 method;
Figure 6 is a flow chart of processing steps performed by the renewal reminder system in concatenating the reminder table generated in Figure 5, and which forms part of the Figure 4 method;
Figure 7 is a flow chart of processing steps performed by the renewal reminder system in generating an electronic, e.g. SMS or Email message, based on the concatenated data produced in the Figure 6 process, and which forms part of the Figure 4 method;
Figure 8 is an example client table, which forms part of a database used by the renewal reminder system;
Figure 9 is a pet table, which forms part of the database;
Figure 10 is an invoice table, which forms part of the database;
Figure 11 is a reminder system table, which forms part of the database;
Figure 12 is a reminder table when sorted, which is generated using data stored in the database;
Figure 13 is a first concatenated version of the reminder table, which is generated by the renewal reminder system;
Figure 14 is a second concatenated version of the reminder table generated by the Figure 13 process, which is generated by the renewal reminder system;
Figure 15 is a storage container configured to work with the renewal reminder system;
Figure 16 is a schematic block diagram of functional modules of the storage container in Figure 15; and
Figure 17 is a reminder system table, which forms part of the database, storing data associated with an external unit, e.g. the storage container of Figure 15.

### Detailed Description of Preferred Embodiments

Embodiments herein relate to a reminder system and method for any renewable or re-orderable product or service. For the avoidance of doubt, the pair of terms renewable and re-orderable, and the pair of terms product and service, are considered synonymous.

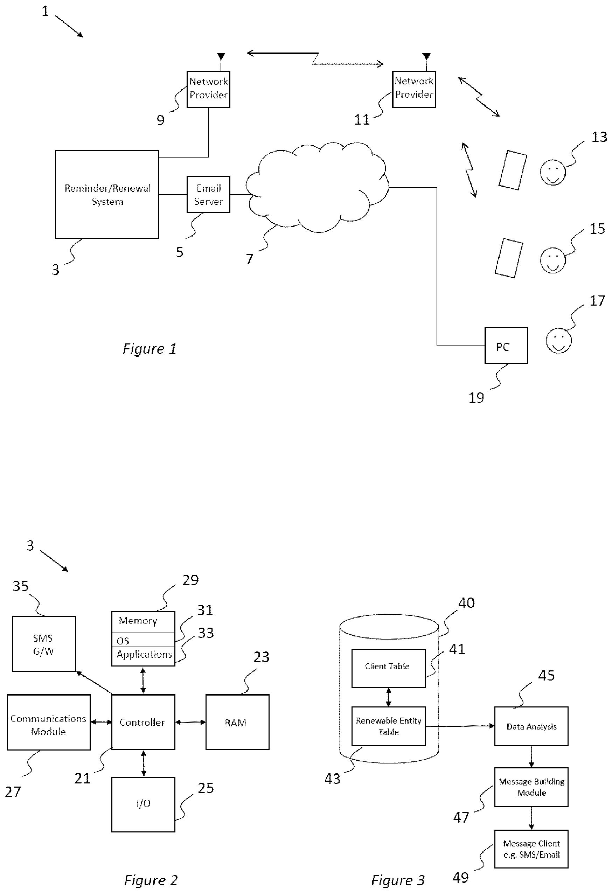

Referring to Figure 1, a reminder system 1 comprises a Renewal Reminder System (RRS) 3 at a first location, which is connected to an Email server 5 and to a mobile communications network provider 9. The Email server 5 is connected to the Internet 7 in the conventional way, and, as will be appreciated, handles the queuing, transmission and receiving of emails. The network provider 9 handles voice and data calls, including the transmission and reception of Short Messaging Service (SMS) text messages. Another network provider 11 is shown located at another location to indicate the onwards transmission of such SMS messages to users 13 and 15, or more particularly, their mobile terminals as shown. A further user 17 is shown with an associated computer terminal 19, which is able to receive email messages sent via the email server 5 and Internet 7 in the usual way.

Referring to Figure 2, a schematic diagram of the components of the RRS 3 is shown. The RRS 3 has a controller 21, RAM 23, input and output devices 25 typically in the form of a keyboard, display and mouse controller, a communications module 27, memory 29 and a SMS gateway 35. The controller 21 is connected to each of the other components in order to control operation thereof.

The memory 29 may be a non-volatile memory such as read only memory (ROM) a hard disk drive (HDD) or a solid state drive (SSD). The memory 29 stores, amongst other things, an operating system 31 and may store software applications 33. The RAM 23 is used by the controller 21 for the temporary storage of data. The operating system 31 may contain code which, when executed by the controller 21 in conjunction with RAM 23, controls operation of each of the hardware components of the RRS 3.

The controller 21 may take any suitable form. For instance, it may be a microcontroller, plural microcontrollers, a processor, or plural processors.

The RRS 3 may be a personal computer (PC), a server, a laptop or any other device capable of running software applications. The communications module 27 enables the RRS 3 to engage in wireless data communications, e.g. cellular communications and/or Wi-Fi. The SMS gateway 35 is a device configured to send SMS text messages over a cellular communications network, e.g. a GSM or UMTS network, and can be purchased off-the-shelf. As will be appreciated, the SMS gateway will include, usually, a Subscriber Identity Module (SIM) card to enable access to the network. In the context of Figure 1, the SMS gateway 35 is connected to the network provider 9 over a wireless connection. Typically, SMS messages are packaged with a 160 character limit and therefore messages passed by the RRS 3 to the gateway 35 should be prepared in some way in order to ensure required reminder information can be conveyed in a single message to avoid excessive data use.

As well as storing the operating system 31 and software applications 33, the memory 29 may also store programs such as email clients and web browsers. One of said software applications 33 stored on memory 29 is a dedicated RRS application configured to generate periodic reminder messages for sending to the users 13, 15, 17 relating to a renewable product or service. In the example below, a veterinary practice uses the RRS 3 to send emails and SMS messages relating to the re-ordering of medicines and/or treatments.

Referring to Figure 3, functional modules on the memory 29 are shown, these being part of the RRS application. A database 40 is provided which stores sets of data used by the RRS application. One database table is a client table 41, and another is a renewable entity table 43. The RRS application performs the functions of data analysis 45, message building 47 and messaging 49 using a SMS and/or email client.

In overview, the client table 41 is a list of the users 13, 15, 17 or clients of the service provider, e.g. the veterinary practice. This table 41 may be provided as part of the RRS application, or be part of an existing CRM package with data being exported therefrom to the RRS application. Typically, the client table 41 includes user names, addresses, and contact information such as email and telephone (landline and mobile) numbers. It will also usually include details of which renewable products/services the client requires.

The renewable entity table 43 is a list of renewable entities, e.g. medicines and/or treatments, together with a renewal profile indicating preferences or features used to determine when renewal reminders should be generated. Again, this table 41 may be provided as part of the RRS application, or be a separate package with data being exported therefrom to the RRS application.

The client and renewable entity tables 41, 43 are collectively used by the RRS application to indicate relationships, i.e. which clients have which renewable entity or entities and when renewal reminders should be issued to individual clients based on a reference date, e.g. when the user purchased the product. In reality, one or both tables may be formed by multiple tables which are linked in the manner of a relational database, as is the case in the example below.

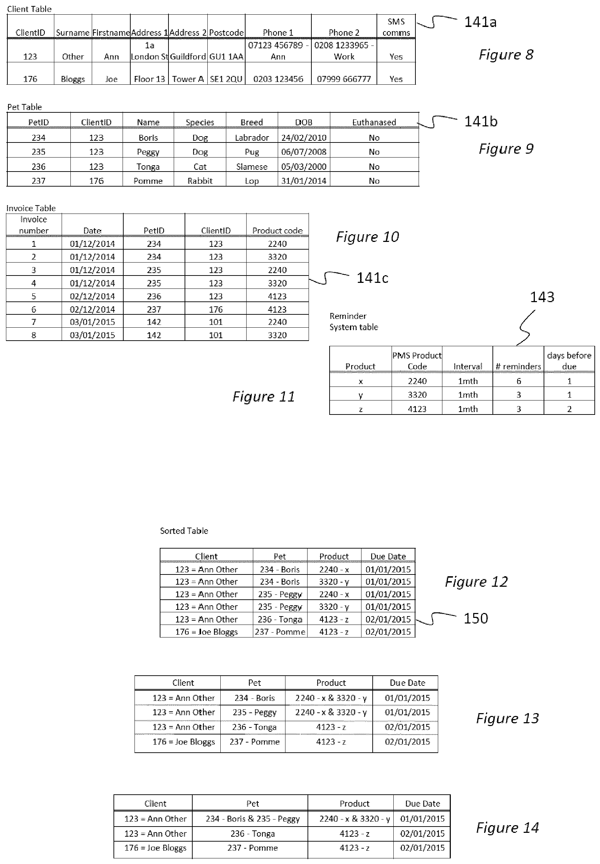

Figure 8 is an example client table 141a for a veterinary practice. The table 141a includes a client ID which is unique to each client. Against each ID is the client surname, first name, address fields, a mobile telephone number and a landline telephone number, as well as a flag which indicates whether or not SMS reminders are applicable. Related to this is a pet table 141b, as shown in Figure 9. The pet table 141b details the pet or pets associated with each client in the client table 141a. Figure 10 is an invoice table 141c which stores a list of invoices for renewable products/services indicating a product code, the client and pet IDs from the Figure 8 and 9 tables, and a reference date which is the date the product was purchased.

In this context, therefore, Figures 8, 9 and 10 provide the client table 41 indicated in Figure 3. Collectively, these tables 141a-c indicate on a client basis, which pets they own, which medicines or treatments each pet is using, and when purchases were made.

Figure 11 is an example reminder system table 143 which stores for each of the renewable products provided by the service provider a renewal profile. In this case, three veterinary products are indicated, namely x, y and z. An interval date is given (in each case, 1 month) which indicates how long the treatment will last before renewal or reordering is needed. A field is provided indicating a number of reminders needed. Finally, a 'days before due' field is provided to indicate how many days before the renewal a reminder should be issued to the client. The reminder system table 143 is therefore linked to the client tables 141a-c by the product code, and can be tailored by the service provider to ensure that appropriate reminders will be issued.

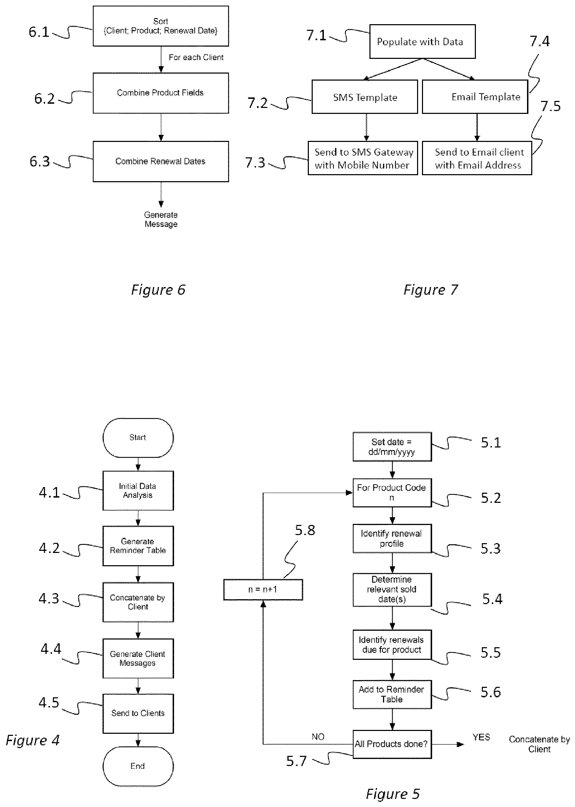

Referring back to Figure 4, the processing steps performed by the RRS application are shown. Each will be introduced, and the tables of Figures 8 to 11 used to provide a practical context. The RRS application is run, for example, automatically without human intervention at a predetermined time each day.

In a first step 4.1 initial data analysis is performed. This can involve removing inappropriate data from the client and pet tables 141a, 141b, e.g. deceased pets, deactivated clients. Data is cleaned, for example by removing non-relevant data stored within telephone number fields. Data can be sorted, for example by searching all fields to ensure a mobile telephone number is present, to avoid sending SMSs to landlines. Treatments can be grouped, e.g. so that treatments that have been superseded by a more recent sale is or are ignored, to avoid sending incorrect messages. This is preferably achieved through the use of multiple level treatment categories, enabling the RRS to interpret when a drug has multiple modalities. For example, a flea treatment (ectoparasiticide) sale should supersede an earlier ectoparasiticide sale. For example, a combined flea and worming treatment (end-ectoparasiticide) sale must supersede an earlier endoparasiticide or ectoparasiticide sale.

In a second step 4.2, a reminder table is generated. In this step, the RRS generates on a client by client basis a list of reminders calculated as being required for issue on that date, taking the data from the client and renewable entity tables 41, 43. In the present embodiment, this involves the following process which is further illustrated by the process of Figure 5.

Referring to Figure 5, in a first step 5.1, the current date is taken. In step 5.2 the next product code n is taken, and in step 5.3 the renewal profile taken from the table 143. From the renewal profile, particularly the interval and days before due fields, a relevant sold date or dates calculation is performed in step 5.4 to determine (based on today's date) what the 'sold' date is in order for the product to require a renewal reminder today.

So, for a current date of 31/12/2014, product x will have a relevant 'sold' date of 1/12/2014 because the interval period is one month (meaning the product will expire on 1/1/2015) and the days before due period is one day. In the case of product z, the relevant 'sold' date will be 2/12/2014 because the days before due period is two days.

In step 5.5, the list of clients is generated for which a renewal reminder is determined as due today from the previous step, for the current product n. For the first iteration for product x, this means that the client 123=Ann Other, who has two pets Boris and Peggy using product x for which a renewal reminder is due today, is added to the client table in step 5.6. In step 5.7 it is determined whether all products have been analysed. If so, the process ends. If not, the next product (y) is analysed, as indicated in the iteration step 5.8.

Figure 12 shows a completed reminder table 150 sorted by client, using the Figure 8 to 11 example data sets. Each of the entries has been determined as requiring a renewal reminder today, as should be clear.

Returning to Figure 4, in step 4.3, the reminder table 150 is concatenated for the purpose of consolidating or reducing the number of entries in preparation for making the reminder message smaller in terms of length and therefore data required to send. In a first concatenation step, the pet name is used as the common factor, with multiple products being added to the product field. In a second concatenation step, the client is used as the common factor, with multiple pets having the same treatments being combined into the pet field. A further step can be used to combine dates. Figures 13 and 14 show concatenated versions in relation to the specific example.

This process is indicated generally in Figure 6, steps 6.1, 6.2 and 6.3 with client the initial common factor.

Concatenation can be performed in different ways, but it will be appreciated that where a message template will be used to generate the reminder message, e.g. of the form:
<Pet(s)> are due <Product(s)> on <Date(s)>....... please call the surgery on 01234 56789 to reorder
then concatenation into common sets of data is useful to minimise message length.

So, instead of sending to the client Ann Other the following:
Boris is due product x on 1/1/15, please call the surgery on 01234 56789 to reorder; Boris is due product y on 1/1/15, please call the surgery on 01234 56789 to reorder; Peggy is due product x on 1/1/15, please call the surgery on 01234 56789 to reorder; Peggy is due product y on 1/1/15, please call the surgery on 01234 56789 to reorder; Tonga is due product z on 2/1/15, please call the surgery on 01234 56789 to reorder,
the following message can be generated:
Boris & Peggy is/are due products x & y on 1/1/15, Tonga is due product z on 2/1/15, please call the surgery on 01234 56789 to reorder.

The latter is obviously preferable for SMS reminder messages, because it clearly fits within the 160 character limit whereas the first one does not. Thus, data transmission and processing at the receiving end is minimised.

Returning to Figure 4, step 4.4 involves generating reminder messages for each client for which a reminder is determined as required. Referring to Figure 7, this involves populating a message template with data from the concatenated table. If an SMS reminder message is to be sent, then a SMS template of the above form will be populated in the manner indicated. Similarly, if an Email reminder message is to be sent, the same process applies although in theory a longer message can be produced. In some cases, both an SMS and Email reminder will be sent. In populating the template to produce a message, verb declination can be performed as required, i.e. is/are, based on whether one or more products are needed. So, rather than the above format, the message is kept minimal by comprising "Boris & Peggy are due Products x & y on 1/1/15 ..."

Finally, in step 4.5, the reminder message(s) is/are sent to the client(s) using, e.g. the SMS gateway and/or an Email client.

The above system and process is configured to run automatically without human intervention on a daily basis, taking data stored in the various database tables. All that is required is for client data to be kept up to date, which is a normal requirement in most CRM situations, and for the product renewal profile to be set as and when a new product is being used.

Although explained in the context of a veterinary practice, it will be appreciated that the generalised system and method can be used in any practical situation where clients need to be sent renewal reminders in relation to a product or service, e.g. in car servicing, insurance renewals and similar fields.

Key features include the reduction of bandwidth usage in generating a single message covering multiple renewals. The system is arranged so that customers do not receive spam messages. The processing occurs at substantially the same time of day on a daily basis. The dates can be managed appropriately according to the particular length of the month. For example, if the reference date is 31/12 and six reminders are needed, the system will send reminders on 30/1 for 31/1, 27/2 for 28/2, 30/3 for 31/3, 29/4 for 30/4 and so on. The system also caters for leap years and deals with dates accordingly and appropriately.

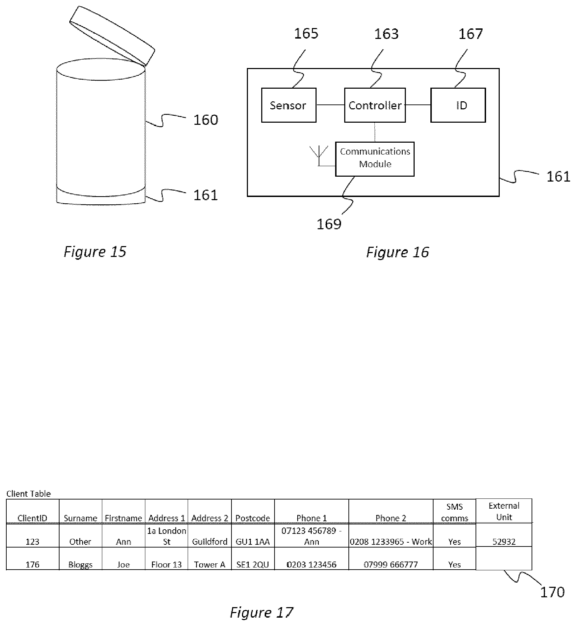

Referring now to Figures 15 and 16, an add-on module for use with the RRS 3 is shown, in the form of a storage container 160 for a renewable product, e.g. pet food or other loose-form product. The storage container 160 may take the form of a stand-alone bucket or bin with a lid. The container 160 employs a sensing system 161 for measuring an indication of the quantity of product stored inside it. In this case, the sensing system 161 is a digital weighting system, e.g. a set of digital scales located at the base of the container 160.

Referring specifically to Figure 16, the sensing system 161 comprises in terms of its functional electronic components a controller 163, a sensor 165, an ID module 16 and a communications module 169. The controller 163 may take any suitable form. For instance, it may be a microcontroller, plural microcontrollers, a processor, or plural processors. The sensor 165 is a set of digital weighting scales. The ID module 16 stores a unique identifier for identifying the container 160 to a remote system, i.e. the RRS 3. The communications module 169 is a networking module configured to communicate with a local Wi-Fi module, e.g. a modem or router located in a user's home, to provide access to the Internet and ultimately the RRS 3 over the Internet. Power to the container 160 may be by means of a battery or mains power.

In use, a client of the RRS 3 can link the storage container 160 to the RRS 3 via their existing account, or through creating a new account. Initially, the client identifies the unique ID 167 to the RRS 3, which may be performed manually, e.g. by telephone or email to the RRS 3 administrator, or through the Internet. In the latter situation, the user can through a local computer terminal, smartphone or tablet obtain the ID 167 and communicate it to the RRS 3 using an Application (App) or web portal associated with the RRS 3. The container 160 may comprise a 'pairing' button or switch for this purpose, which makes the ID 167 available through Wi-Fi or Bluetooth for detection by the local computer terminal. Other methods may be used. At the RRS 3, the message containing the relevant Client ID and the unique container ID 167 are associated and stored together in an updated version of the client table 170, as shown in Figure 17. One or more "External Unit" fields may be created for this purpose. Here, the client ID 123 has a paired external unit with container ID "52932".

An indication of the product being stored in the container can also be sent over, and for this purpose this information is also stored, e.g. in an updated version of the reminder system table shown in Figure 11.

The sensing system 161, particularly the controller 163, is configured through software or firmware to detect the condition whereby the substance it stores, e.g. dog food, drops below a predetermined threshold, in this case weight. This is of course indicative that more food is required imminently. In this condition, the controller 163, through the communications module 169, automatically sends a data message over the Internet to the RRS 3, which contains the unique ID 167 and data indicating the low weight / renewal requirement. This, through the client table 170 is used by the RRS 3 to identify the user, and, through the reminder system table, or another table, the product code requiring renewal.

Responsive to receiving this message, the RRS 3 works as before to add the relevant product code to the reminder table(s) indicated in Figures 12 to 14, sorted and concatenated as before. Therefore, at the next run of the RRS 3, most likely the next day, the client is sent an email or SMS indicating the need to replenish and reorder the identified product, which message will be concatenated with any other product that requires renewal as before. Re-ordering can be made via a telephone call or activable link within the message to initiate the re-ordering.

As a refinement, the controller 163 may be configured to send a regular message over the Internet to the RRS 3 which indicates the weight of the product as it reduces over time. In this way, the RRS 3 can be configured to determine the amount, or average amount, of food that is being taken from the container 160 on a daily or other time-frame, and therefore is able to more accurately predict in advance when a re-order is required. Firmware or software associated with the controller 163 is arranged for this purpose.

It will be appreciated that the above described embodiments are purely illustrative and are not limiting on the scope of the invention. Other variations and modifications will be apparent to persons skilled in the art upon reading the present application.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

## Claims

1. A computer-implemented system for generating renewal messages comprising:
at least one container comprising:
a storage component for storing a stored product;
a communications module for wirelessly connecting to a network; and
a sensor component for identifying the stored product and a quantity of the stored product when present in the storage component and outputting a signal to the network using the communications module, the signal containing one or more of the identification of the stored product, an indication of the stored product quantity, and an indication of whether the quantity is below a predetermined threshold amount;
at least one memory, storing:
(i) for each of a plurality of products, a type of treatment the product is used for and a renewal profile including at least a renewal period for the products; and
(ii) for each of a plurality of customers, a customer data including a contact data, one or more product codes for each product used by the customer, a reference date corresponding to a purchase date of each one of the products used by the customer, and a storage container identifier identifying the at least one container; and
a processor-executable software stored in a computer-readable media adapted to, for each of the plurality of customers:
receiving the signal from the network;
determining based on at least the stored reference dates and product codes whether any one of the products used by the customer is superseded by comparing the products codes and the corresponding reference dates of the products used, to identify any one or more products used that are the same as another product used that has an older reference date;
determining based on at least the stored treatment type whether any one of the products used by the customer is superseded by comparing the types of treatment, to identify any one or more products used that have a single treatment type that is the same as another product used that has two or more treatment types where one of the at least two or more treatment types is the same as the single treatment type;
calculating a renewal date for each of the products used by the customer using the reference date for each product and the renewal period;
except for any one of the superseded products, generating a renewal message using a predetermined SMS template for (i) each of the products when the current date is within a predetermined period of time before the calculated renewal date, and (ii) each of the products when the quantity is below the predetermined threshold amount; and
if more than one renewal messages are generated for each customer, concatenating the more than one renewal messages into a single renewal message to reduce the number of characters below a predefined character limit; and
sending an SMS message based on the concatenated renewal message using an SMS gateway.

2. A system according to claim 1, wherein the renewal message is concatenated to consolidate or reduce the number of entries in preparation for making the reminder message smaller in terms of character length and data required to send the message, and also that the message fits within a predefined characters' limit; and
wherein the renewal message is concatenated in terms of character length so that the message fits within a predefined characters' limit of a computer-readable code.

3. A system according to any preceding claim, wherein the customer data further includes an SMS contact data,
wherein the processor-executable software is further configured to converting the renewal message into a single SMS message using the SMS contact data; and
wherein the SMS message template comprises one or more data entry fields.

4. A system according to any preceding claim, wherein the renewal profile for a product stored in the at least one memory comprises:
a product identifier;
the renewal date; and
an advance period, and
wherein the processor-executable software is further configured to, for each product,
using the renewal date and the advance period for the product to determine a comparison reference date; and
identifying from the customer data those customers having the associated product identifier and a match between the comparison references date and the reference date, where after matches are added to a customer list, including the relevant product identifier; and
wherein the reference date is stored on an electronic records/invoicing system for different than the at least one memory; and
wherein the processor-executable software is further adapted to receiving the reference date by initiating a periodic data request to the electronic records /invoices system.

5. A system according to any preceding claim, wherein the processor-executable software is further configured to, responsive to receiving an electronic message initiated by said storage container, including in said generated renewal message, a reference to a product renewal for the storage product associated with the storage container.

6. A system according to any preceding claim, wherein the processor-executable software is further configured to,
receiving a pairing message containing an indication of a customer identifier and the storage container identifier, and
updating the customer data to include the storage container identifier, wherein the pairing message further contains a product identifier or code, which is at least one of updated to the customer data or associated with it.

7. A computer-implemented method for generating renewal messages comprising:
storing in at least one memory device:
(i) for each of a plurality of products, a type of treatment the product is used for and a renewal profile including at least a renewal period for the product;
(ii) for each of a plurality of customers, customer data, including a contact data, one or more product codes for each product used by the customer, a reference date corresponding to a purchase date of each one of products used by the customer, and a storage container identifier identifying at least one container;
providing a processor-executable software stored in a media device, the software adapted to:
determining based on at least the stored reference dates and product codes whether any one of the products used by the customer is superseded by comparing the products codes and the corresponding reference dates of the products used, to identify any one or more products used that are the same as another product used that has an older reference date;
determining based on at least the stored treatment type whether any one of the products used by the customer is superseded by comparing the types of treatment, to identify any one or more products used that have a single treatment type that is the same as another product used that has two or more treatment types where one of the at least two or more treatment types is the same as the single treatment type;
calculating a renewal date for each of the products used by the customer using the reference date for each product;
except for any one of the superseded products, generating a renewal message using a predetermined SMS template for (i) each of the products when the current date is within a predetermined period of time before the calculated renewal date, and (ii) each of the products when the quantity is below a predetermined threshold amount; and
if more than one renewal messages are generated for each customer, concatenating the more than one renewal messages into a single renewal message to reduce the number of characters below a predefined character limit; and
sending an SMS message based on the concatenated renewal message using an SMS gateway.

8. A method according to claim 7, wherein the renewal message is concatenated to consolidate or reduce the number of entries in preparation for making the reminder message smaller in terms of character length and data required to send the message, and also that the message fits within a predefined characters' limit; and
wherein the renewal message is concatenated in terms of character length so that the message fits within a predefined characters' limit of a computer-readable code.

9. A method according to claim 7 or claim 8, wherein the renewal profile for each of the plurality of products comprises:
a product identifier;
a renewal period; and
an advance period,
and wherein generating the renewal profile comprises, for each product,
using the renewal period and advance period for the product to determine a comparison reference date; and
identifying from the customer data those customers having the associated product identifier and a match between the comparison reference date and the reference date, where after matches are added to the customer list, including the relevant product identifier.

10. A method according to any one of claims 7 to 9, wherein the reference date is a date when the product was purchased or a date on an invoice for the product, and wherein the reference date is stored on an external electronic records/invoicing/reminder system; and
wherein the reference date is received by initiating a periodic data request to the external electronic records system.

11. A method according to any one of claims 7 to 10, further comprising:
providing a SMS message template including a standard message and one or more data entry fields into which the processor automatically populates renewal data; and
wherein a plurality of templates is provided based on context.

12. A method according to any one of claims 7 to 11, further comprising storing, as part of, or associated with, the customer data, an identity of a storage container associated with one or more respective customers, and responsive to receiving an electronic message initiated by said storage container to include in said generated message a reference to a product renewal, which product is associated with the customer or storage container.

13. A method according to any one of claims 7 to 12, further comprising receiving a pairing message received from a remote location containing an indication of a customer identifier and a storage container identifier, and updating the customer data automatically to include the storage container identifier, wherein the pairing message further contains a product identifier or code, which is at least one of updated to the customer data or associated with it.

14. A method according to any one of claims 7 to 13, wherein the customer data indicates (i) a sale of the product having multiple modalities that supersedes another product, and (ii) a sale of the another product that is superseded by the product having multiple modalities.

15. A storage container for containing material, said storage container comprising a sensor for detecting or estimating the quantity of material held within it, a data identifier associated with the container, and a wireless transmitter for transmitting a message indicative of the data identifier and the quantity of material held within the container for use with system according to any of claims 1 to 6, and wherein the sensor comprises a weighing scale.
